(19) Europäisches Patentamt
European Patent Office
Office européen des brevets

(11) Veröffentlichungsnummer : **0 220 587**
**B1**

(12) # EUROPÄISCHE PATENTSCHRIFT

(45) Veröffentlichungstag der Patentschrift : **24.01.90**

(51) Int. Cl.[5] : **B 01 J 23/44**, B 01 J 37/02, C 07 C 29/20

(21) Anmeldenummer : **86114130.7**

(22) Anmeldetag : **13.10.86**

(54) **Trägerkatalysatoren und Hydrierverfahren unter Verwendung dieser Trägerkatalysatoren.**

(30) Priorität : **26.10.85 DE 3538129**

(43) Veröffentlichungstag der Anmeldung : **06.05.87 Patentblatt 87/19**

(45) Bekanntmachung des Hinweises auf die Patenterteilung : **24.01.90 Patentblatt 90/04**

(84) Benannte Vertragsstaaten : **DE FR GB IT NL**

(56) Entgegenhaltungen :
**EP-A- 0 012 153**
**EP-A- 0 013 286**
**DE-A- 2 045 882**
**GB-A- 2 035 120**
**US-A- 3 271 327**
**US-A- 4 203 923**

(73) Patentinhaber : **BAYER AG**
**D-5090 Leverkusen 1 Bayerwerk (DE)**

(72) Erfinder : **Medem, Harald, Dr.**
**Scheiblerstrasse 85**
**D-4150 Krefeld (DE)**
Erfinder : **Birkenstock, Udo, Dr.**
**Schneppersdelle 2**
**D-4030 Ratingen 8 (DE)**
Erfinder : **Schmidt, Herbert**
**Im Hederichsfeld 52**
**D-5090 Leverkusen 3 (DE)**
Erfinder : **Lachmann, Burkhard, Dr.**
**Lettweg 3**
**D-4005 Meerbusch (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

## Beschreibung

Die vorliegende Erfindung betrifft verbesserte Trägerkatalysatoren, deren Herstellung und ein Verfahren zur Hydrierung von Phenolen zu den entsprechenden Cyclohexanolen unter Verwendung solcher Trägerkatalysatoren.

Unter Trägerkatalysatoren werden Zusammensetzungen verstanden, die katalytisch aktive Substanzen, z. B. Metalle als solche oder in Form von Verbindungen, auf Trägermaterialien, z. B. Siliciumdioxid, Aluminiumoxid, Magnesiumund Aluminiumsilikaten oder Carbonaten, enthalten. Eine besondere Bedeutung haben Katalysatoren erlangt, die Edelmetalle auf Trägern mit einer niedrigen BET-Oberfläche, z. B. einer solchen von weniger als 50 $m^2/g$ enthalten. Diese können z. B. durch Tränken des Trägermaterials mit einer wäßrigen Lösung von Edelmetallsalzen und anschließender Reduktion erhalten werden.

Beispielsweise werden in der DE-A-2 045 882 palladiumhaltige Trägerkatalysatoren und deren Verwendung bei der Hydrierung von Phenol zu Cyclohexanon beschrieben, zu deren Herstellung hochgeglühte Aluminium-Spinell-Trägermaterialien, wie Lithium-, Magnesium-, Kobalt-, Mangan- oder Zink-Spinelle, eingesetzt werden. Im allgemeinen sind bei solchen Katalysatoren die katalytisch aktiven Substanzen unregelmäßig über das gesamte Trägermaterial verteilt. Als Folge davon können beim technischen Einsatz dieser Katalysatoren unerwünschte Aktivität-, Selektivität- und Standzeit-Unterschiede auftreten. Ferner kommt es häufig vor, daß die in der Nähe des Zentrums eines Trägerteilchens vorhandenen Substanzen die zu katalysierende Reaktion entweder gar nicht oder nur in untergeordnetem Maße beeinflussen. Bei wertvollen katalytisch aktiven Substanzen, z. B. bei Edelmetallen und deren Verbindungen, führt dies zu unnötig hohen Katalysatorkosten.

Es sind deshalb schon Versuche unternommen worden, Katalysatoren herzustellen, bei denen Trägermaterialien mit kleiner BET-Oberfläche die katalytisch aktiven Substanzen nur in einem schmalen, äußeren Bereich der Trägerteilchen enthalten. Verfahren dieser Art werden beispielsweise beschrieben in den DE-Offenlegungsschriften 1 944 933, 2 517 313, 2 317 536 und 2 715 094 den US-PS' en 3 271 327 und 2 946 829 und der GB-Patentschrift 1 283 737.

Alle diese Verfahren und die dabei erhältlichen Katalysatoren sind jedoch immer noch mit erheblichen Nachteilen behaftet. So sind die zur Katalysatorherstellung eingesetzten Trägermaterialien aufgrund ihrer Oberflächenbeschaffenheit im allgemeinen nicht inert und bedingen daher, insbesondere bei großen inneren Oberflächen, bei ihrem technischen Einsatz in der Regel die Bildung unerwünschter Nebenprodukte. Ein weiterer Nachteil bei der Verwendung der so hergestellten Katalysatoren besteht darin, daß es bei den auf der äußeren Oberfläche des Trägerkorns in sehr hoher Konzentration abgeschiedenen Edelmetallen sehr leicht zu Abriebverlusten und zu Vergiftungen kommt. Es werden deshalb in vielen Fällen nur ungenügende Katalysatorstandzeiten erreicht.

In der EP-A 0 012 153 werden u. a. Trägerkatalysatoren beschrieben, bei denen inertes Trägermaterial mit einer BET-Oberfläche von weniger als 20 $m^2/g$ mit der Lösung einer Base bis zur Sättigung getränkt, anschließend bis zu Gewichtskonstanz getrocknet, dann mit einer Metallsalzlösung ebenfalls bis zur Sättigung getränkt und schließlich zum Metall reduziert wurde, wobei die Base in einer solchen Menge aufgebracht wurde, daß vor der Tränkung mit der Metallsalzlösung im Trägermaterial 0,01 bis 50 Grammäquivalente Base pro Grammäquivalent Metall enthalten sind. Nach diesem verfahren hergestellte Katalysatoren enthalten die katalytisch aktiven Substanzen in einer engen ringförmigen Zone im Inneren des inerten Trägermaterial, d. h. direkt unter der Oberfläche der Trägerteilchen, wodurch die katalytisch aktiven Substanzen einerseits weitgehend vor Vergiftungen und Verlusten durch Abrieb geschützt sind, andererseits aber in einem Bereich des Trägermaterials abgelagert sind, der von den Reaktionskomponenten noch gut erreicht werden kann.

Beim Einsatz eines derartigen Trägerkatalysators, enthaltend 9 g Palladium pro Liter $\alpha$-$Al_2O_3$-Träger mit einer BET-Oberfläche von 9,8 $m^2/g$, zur Hydrierung von Phenol wurde gemäß Beispiel 72 der EP-A-0 012 153 ein Reaktionsprodukt erhalten, das 96,2 Gew.-% Cyclohexanon und 3,7 Gew.-% Cyclohexanol enthielt.

Häufig ist jedoch die Bildung von Cyclohexanolen erwünscht, da auch Cyclohexanol und Derivate davon von großtechnischer Bedeutung sind. Cyclohexanol selbst wird z. B. als Ausgangsprodukt zur Herstellung von Adipinsäure benötigt, einem ebenfalls wichtigen Grundstoff der chemischen Industrie (siehe z. B. Kirk-Othmer, Encyclopedia of Chemical Technology, 2nd edition, Vol. 6, Seite 685). Auch verschiedene Ester des Cyclohexanols haben eine großtechnische Bedeutung, beispielsweise Dicyclohexylphthalat und -adipat, als Weichmacher für Kunststoffe, Cyclohexylacetat als Lösungsvermittler und Cyclohexylacrylat als Comonomer für Polymerisationen. Andere Cyclohexanolderivate, z. B. Methylcyclohexanol und Trimethylcyclohexanol werden als Lösungs- und Emulgiermittel in der Lack- und Textilindustrie eingesetzt (siehe z. B. Ullmanns Enzyklopädie der techn. Chemie, 4. Aufl., Band 9, S. 692-693). Schließlich ist Perhydrobisphenol eine wichtige Ausgangskomponente bei Polykondensationen (siehe z. B. Ullmanns Enzyklopädie der techn. Chemie, 4. Aufl. Band 7, S. 229).

Es wurden nun Trägerkatalysatoren gefunden, die erhältlich sind, indem man nacheinander

a) ein inertes Trägermaterial mit einer BET-Oberfläche von weniger als 20 $m^2/g$ mit einer Base bis zur Sättigung tränkt,

b) bis zur Gewichtskonstanz trocknet,

c) mit einer Palladiumsalzlösung bis zur Sättigung tränkt,

d) mit Wasser wäscht, bis keine Ionen aus den bis jetzt verwendeten Verbindungen mehr im Waschwasser nachweisbar sind und

e) trocknet,

die dadurch gekennzeichnet sind, daß man danach

f) so viel einer Base aufbringt, daß der Katalysator 0,01 bis 50 Grammäquivalente Base pro Grammäquivalent Palladium enthält,

g) bis zur Gewichtskonstanz trocknet und

h) zu einem beliebigen Zeitpunkt nach Durchführung des Schrittes c) und vor der Durchführung der mit dem Katalysator zu katalysierenden Reaktion die abgeschiedenen Palladiumverbindungen zum Metall reduziert, bei Durchführung dieser Reduktion nach dem Schritt f) unter Bedingungen, bei denen die im Schritt f) aufgebrachte Base auf dem Katalysator verbleibt.

Trägerkatalysatoren, die durch Ausführung der Schritte a) bis e) erhältlich sind, sind bekannt und z. B. in der EP-A-0 012 153 detailliert beschrieben. Im folgenden werden deshalb die Schritte a) bis e) nur kurz erläutert.

Bei dem im Schritt a) zu verwendenden inerten Trägermaterial kann es sich beispielsweise um Metalloxide, Silikate, Spinelle, Carbide, Carbonate oder Mischungen davon handeln. Bevorzugte Trägermaterialien sind Aluminiumoxide, Siliciumdioxide, Siliciumdioxid-Aluminiumoxid-Gemische, amorphe Kieselsäuren, Kieselgure, Barium-, Strontium- oder Calciumcarbonate, Mischungen derselben, die gegebenenfalls zusätzlich Siliciumdioxide oder Aluminiumoxide enthalten, Titanoxide, Zirkonoxide, Magnesiumoxide, Magnesiumsilikate, Zirkonsilikate, Magnesium-Aluminium-Spinelle, Siliciumcarbide, Wolframcarbide, Mischungen von Siliciumcarbiden mit Siliciumdioxiden oder beliebige Mischungen der vorgenannten Materialien. Besonders bevorzugt ist Aluminiumoxid, insbesonders $\alpha$-$Al_2O_3$. Die Trägermaterialien können in den verschiedensten Formen zur Anwendung kommen, z. B. als Kugeln, Granulate, Extrudate, Tabletten, Sattelkörper, Rohrabschnitte, Bruchstücke und/oder Wabenkeramik. Vorzugsweise weist das inerte Trägermaterial eine BET-Oberfläche von weniger als 10 $m^2$/g auf.

Bei der im Schritt a) zu verwendenden Base kann es sich beispielsweise um ein Oxid, Carbonat, Hydrogencarbonat, Hydrogenphosphat, Hydroxid, Alkoxid, Formiat, Alkalisilikat, Alkalialuminat oder um Mischungen davon handeln. Bevorzugt sind Alkalihydroxide und/oder Alkalicarbonate.

Bevorzugt werden die Basen in gelöster Form eingesetzt, wobei Wasser oder organische Lösungsmittel, z. B. Alkohole oder Ketone, als Lösungsmittel verwendet werden können. Bevorzugt sind hier wäßrige Lösungen von Alkalihydroxiden.

Die Base kann im Schritt a) z. B. in einer solchen Menge angewendet werden, daß nach dem Schritt a) im Trägermaterial 0,01 bis 50 Grammäquivalente Base, vorzugsweise 0,5 bis 20 Grammäquivalente Base, pro Grammäquivalent nach dem Schritt c) im Trägermaterial vorhandenes elementares oder gebundenes Palladium vorliegen.

Die Trocknung im Schritt b) kann beispielsweise bei 50 bis 200 °C in einem Trockenschrank oder in einem Warmluftstrom erfolgen.

Im Schritt c) wird vorzugsweise eine Lösung eines handelsüblichen einfachen oder komplexen Palladiumsalzes verwendet. Bevorzugt sind wäßrige Lösungen von $Na_2[PdCl]_4$, $PdCl_2$ und/oder $Pd(NO_3)_2$. Die Konzentration einer solchen Lösung kann z. B. so bemessen werden, daß mit einer einmaligen Tränkung so viel palladiumverbindungen auf den Träger aufgebracht werden, daß er danach 1 bis 20 g Palladium in elementarer oder gebundener Form pro Liter enthält.

Die Wäsche des Schrittes d) dient dazu noch auf dem Träger vorhandene lösliche Ionen zu entfernen. Beispielsweise kann es sich dabei um Ionen der eingesetzten Base und/oder um Anionen des eingesetzten palladiumsalzes handeln. Es ist im allgemeinen vorteilhaft zwischen Schritt c) und Schritt d) eine Wartezeit von beispielsweise 2 Stunden bis 3 Tagen einzuhalten.

Die Trocknung des Schritte e) kann wie diejenige des Schrittes b) durchgeführt werden.

Es ist ein wesentliches Merkmal der erfindungsgemäßen Trägerkatalysatoren, daß sie erhältlich sind, indem nach dem Aufbringen von Palladium unter definierten Bedingungen (sieche die Schritte d) und e)) eine bestimmte Menge Base nachdotiert wird, die bis zur Verwendung des Katalysators (einschließlich) auf ihm verbleibt.

Für den Schritt f) kommen als Basen beispielsweise Oxide, Hydroxide, Alkoxide, Carbonate, Hydrogencarbonate, Hydrogenphosphate, Formiate, Silikate und/oder Aluminate in frage, z. B. solche von Alkali- und/oder Erdalkalimetallen, besonders bevorzugt von Lithium, Kalium, Magenesium, Calcium, Strontium und/oder Barium. Besonders bevorzugt sind deren Hydroxide und Oxide, wie Lithiumhydroxid, Kaliumhydroxid, Magnesiumoxid, Calciumhydroxid, Strontiumhydroxid und Bariumhydroxid. Ganz besonders bevorzugt ist Bariumhydroxid.

Im Schritt f) wird die Base im allgemeinen in Form einer Lösung durch Tränken aufgebracht. Als Lösungsmittel kommen Wasser oder organische Lösungsmittel, z. B. Alkohole oder Ketone, in Frage. Bevorzugt ist Wasser. Die Basen können jedoch auch als solche aufgebracht werden, dann vorzugsweise in fein verteilter Form, oder im Gemisch mit einer beliebigen flüssigen Phase, in der die Base nicht oder nicht vollständig löslich sein muß.

Bevorzugt werden im Schritt f) 0,01 bis 50 Grammäquivalente Base pro Grammäquivalent auf dem Katalysator vorhandenes Palladium aufgebracht.

Der Schritt g) kann in der gliechen Weise durchgeführt werden wie die Schritte b) und e).

Bevor der erfindungsgemäße Katalysator verwendet werden kann, sind die auf ihm befindlichen Palladiumverbindungen noch zum Metall zu reduzieren (Schritt h)). Diese Reduktion kann zu einem beliebigen Zeitpunkt nach der Durchführung des Schrittes c) und vor der Durchführung der mit dem Katalysator zu katalysierenden Reaktion erfolgen. Vorzugsweise wird diese Reduktion direkt anschließend an den Schritt c) oder in situ, d. h. unmittelbar vor Durchführung der mit dem Katalysator zu katalysierenden Reaktion, durchgeführt. Diese Reduktion kann auf an sich bekannte Weise und mit für solche Reduktionen üblichen Reduktionsmitteln ausgeführt werden. Geeignete Reduktionsmittel sind beispielsweise Hydrazinhydrat, Wasserstoff, Formaldehyd und Natriumborhydrid. Bevorzugte Reduktionsmittel sind gasförmiger Wasserstoff und wäßriges Hydrazinhydrat.

Da es ein erfindungswesentliches Merkmal ist, daß der fertige Katalysator die im Schritt f) aufgebrachte Base noch enthält, ist darauf zu achten, daß bei Durchführung der Reduktion nach dem Schritt f) die im Schritt f) aufgebrachte Base auf dem Katalysator verbleibt. In solchen Fällen, also z. B. bei der vorerwähnten in-situ-Reduktion, können deshalb keine wäßrigen Lösungen von Reduktionsmitteln eingesetzt werden. Vorzugsweise wird dann mit gasförmigem Wasserstoff reduziert.

Es können auch mehrmalige Behandlungen mit Reduktionsmitteln durchgeführt werden, beispielsweise anschließend an den Schritt c) und in situ (letzteres wie zuvor beschrieben).

Die erfindungsgemäßen Trägerkatalysatoren können für die verschiedensten edelmetallkatalysierten Reaktionen verwendet werden, insbesondere für Hydrierungen und Dehydrierungen. Bei katalytischen Reaktionen können die erfindungsgemäßen Trägerkatalysatoren sowohl in der Sumpfphase als auch in der Rieselphase und in der Gasphase eingesetzt werden. Bevorzugt sind die Rieselphase und die Gasphase. Dabei kann sowohl unter Normaldruck als auch bei Überdruck gearbeitet werden. Katalytische Hydrierungen sind ein bevorzugtes Anwendungsgebiet für die erfindungsgemäßen Trägerkatalysatoren. Sie eignen sich besonders zur Kernhydrierung aromatischer Systeme und zur Hydrierung bestimmter Substituenten, beispielsweise Nitrogruppen an aromatischen Systemen. Die erfindungsgemäßen Trägerkatalysatoren werden bevorzugt bei der katalytischen Hydrierung von Phenol und substituierten Phenolen angewendet, wobei das aromatische System und/oder der Substituent hydriert werden können. Die besonders bevorzugte Verwendung erfindungsgemäßer Trägerkatalysatoren bei der Hydrierung von Phenolen zu Cyclohexanolen ist ein weiterer Gegenstand der vorliegenden Erfindung und wird weiter unten detailliert beschrieben.

Die vorliegende Erfindung betrifft weiterhin ein Verfahren zur Herstellung von trägerkatalysatoren, bei dem man nacheinander

a) ein inertes Trägermaterial mit einer BET-Oberfläche von weniger als 20 $m^2/g$ mit einer Base bis zur Sättigung tränkt,

b) bis zur Gewichtskonstanz trocknet,

c) mit einer Palladiumsalzlösung bis zur Sättigung tränkt,

d) mit Wasser wäscht, bis keine Ionen aus den bis jetzt verwendeten Verbindungen mehr im Waschwasser nachweisbar sind und

e) trocknet,

das dadurch gekennzeichnet ist, daß man danach

f) so viel einer Base aufbringt, daß der Katalysator 0,01 bis 50 Grammäquivalente Base pro Grammäquivalent Palladium enthält,

g) bis zur Gewichtskonstanz trocknet und

h) zu einem beliebigen Zeitpunkt nach Durchführung des Schrittes c) und vor der Durchführung der mit dem Katalysator zu katalysierenden Reaktion die abgeschiedenen Palladiumverbindungen zum Metall reduziert, bei der Durchführung dieser Reduktion nach dem Schritt f) unter Bedingungen, bei denen die im Schritt f) aufgebrachte Base auf dem Katalysator verbleibt.

Die weiter oben beschriebenen geeigneten und bevorzugten Maßnahmen mit denen erfindungsgemäße Trägerkatalysatoren erhältlich sind, sind auch bei dem erfindungsgemäßen Verfahren zur Herstellung von Trägerkatalysatoren geeignet und bevorzugt.

Die vorliegende Erfindung betrifft schließlich noch ein Verfahren zur Hydrierung von gegebenenfalls substituierten Phenolen zu Cyclohexanolen, das dadurch gekennzeichnet ist, daß man es in Gegenwart von Trägerkatalysatoren durchführt, die erhältlich sind, indem man nacheinander

a) ein inertes Trägermaterial mit einer BET-Oberfläche von weniger 20 $m^2/g$ mit einer Base bis zur Sättigung tränkt,

b) bis zur Gewichtskonstanz trocknet.

c) mit einer Palladiumsalzlösung bis zur Sättigung tränkt,

d) mit wasser wäscht, bis keine Ionen aus den bis jetzt verwendeten Verbindungen mehr im Waschwasser nachweisbar sind,

e) trocknet,

f) so viel einer Base aufbringt, daß der Katalysator 0,01 bis 50 Grammäquivalente Base pro Grammäquivalent Palladium enthält,

g) bis zur Gewichtskonstanz trocknet und

h) zu einem beliebigen Zeitpunkt nach Durchführung des Schrittes c) und vor der Durchführung der mit dem Katalysator zu katalysierenden Reaktion die abgeschiedenen palladiumverbindungen zum Metall reduziert, bei Durchführung dieser Reduktion nach dem Schritt f), unter Bedingungen, bei denen die im Schritt f) aufgebrachte Base auf dem Katalysator verbleibt.

In das erfindungsgemäße Hydrierverfahren können beispielsweise Phenols der Formel (I) eingesetzt werden

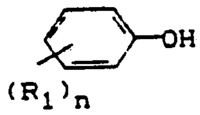 (I)

in der

$R_1$ für $C_1$-bis $C_6$-Alkyl, $C_6$-bis $C_{10}$-Aryl, Hydroxyl, Nitro, Halogen, gegebenenfalls substituiertes Amino, $C_1$- bis $C_6$-Alkoxy oder $C_6$-bis $C_{10}$-Aroxy und

n für Null oder eine ganze Zahl von 1 bis 5 stehen,

wobei jedoch im Falle $R_1$ = Nitro n nur für Null, 1 oder 2 steht.

Halogen kann dabei Fluor, Chlor, Brom oder Jod bedeuten, gegebenenfalls substituiertes Amino beispielsweise eine $NH_2$-Gruppe, die gegebenenfalls ein- oder zweimal mit $C_1$- bis $C_6$-Alkyl oder $C_6$- bis $C_{10}$-Aryl substituiert ist. Sofern mehrere Reste $R_1$ vorhanden sind, d.h. wenn n für 2, 3, 4 oder 5 steht, können diese $R_1$-Reste gleich oder verschieden sein.

Vorzugsweise steht $R_1$ für Methyl, Ethyl, n-Propyl, i-Propyl, n-Hexyl, Phenyl, Hydroxyl, Chlor, Brom, $NH_2$, Methoxy, Ethoxy oder Phenoxy und n für Null, 1 oder 2.

Besonders bevorzugt steht $R_1$ für Methyl in meta- und/oder para Stellung und n für Null oder 1.

Es können beispielsweise auch Phenole der Formel (II) eingesetzt werden

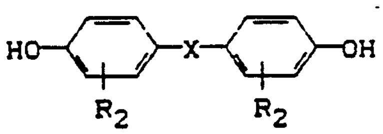 (II)

in der

$R_2$ unabhängig voneinander jeweils für Wasserstoff, $C_1$- bis $C_6$-Alkyl, $C_1$- bis $C_6$-Alkoxy oder Halogen und

X für $-CH_2-$, $-C(CH_3)_2-$, —Cyclohexyl—, —CO—, —S—, $-SO_2-$, —O— oder eine Einfachbindung stehen.

Vorzugsweise steht $R_2$ für Wasserstoff oder Methyl, und X für $-C(CH_3)_2-$.

Der Einsatz von Phenolen der Formel (I) ist gegenüber dem Einsatz von Phenolen der Formel (II) bevorzugt.

Das erfindungsgemäße Hydrierverfahren wird bevorzugt mit solchen Katalysatoren durchgeführt, die erhältlich sind durch die weiter oben (bei der Beschreibung der erfindungsgemäßen Katalysatoren) als vorteilhaft angegebenen Maßnahmen. Besonders bevorzugt werden im erfindungsgemäßen Hydrierverfahren Katalysatoren eingesetzt, die als inertes Trägermaterial $\alpha$-$Al_2O_3$ und als Base Bariumhydroxid enthalten.

Das erfindungsgemäße Hydrierverfahren kann sonst unter üblichen Reaktionsbedingungen und in üblichen Reaktoren durchgeführt werden. Beispielsweise kann man die erfindungsgemäße Hydrierung mit leicht verdampfbaren Phenolen in der Gasphase und in Röhrenreaktoren und mit schwer verdampfbaren Phenolen in der Rieselphase und in Schachtöfen durchführen. Die Reaktionstemperaturen können beispielsweise zwischen 100 und 350 °C liegen. Bevorzugt liegen sie zwischen 120 und 250 °C. Der Druck während der Reaktion kann beispielsweise zwischen 1 und 350 bar betragen. Bei Durchführung der Reaktion in der Gasphase sind dabei im allgemeinen relativ niedrige Drucke, beispielsweise solche im Bereich 1 bis 5 bar, bevorzugt. Bei Durchführung der Reaktion in der Rieselphase sind dabei im allgemeinen relativ hohe Drucke, beispielsweise solche im Bereich 100 bis 350 bar, bevorzugt. Pro Mol des jeweils eingesetzten Phenols werden dem Reaktor im allgemeinen mindestens 2 Mole Wasserstoff zugeführt. Bevorzugt werden 4 bis 12 Mole Wasserstoff pro Mol des jeweils eingesetzten Phenols in den Reaktor eingeleitet. Wenn die erfindungsgemäße Hydrierung in der Gasphase durchgeführt wird kann man beispielsweise so verfahren, daß man das Phenol im Wasserstoff-Einsatz-Strom verdampft und diesen kombinierten Strom dann dem Reaktor zuführt. Sofern in das erfindungsgemäße Hydrierverfahren Katalysatoren eingesetzt werden, bei denen vor der Beaufschlagung mit einem Phenol noch die vorhandenen Palladiumverbindungen reduziert werden müssen, kann dies auf besondere einfache Weise

geschehen, indem man zunächst nur Wasserstoff über den im Reaktor befindlichen Katalysator strömen Läßt und erst, wenn die Palladiumverbindungen reduziert sind, das jeweilige Phenol zuführt.

Die den Reaktor verlassenden Produkte werden im allgemeinen abgekühlt und die hydrierten Phenole in flüssiger oder fester Form abgetrennt. Sie können gegebenenfalls nach üblichen Methoden weiter gereinigt werden, beispielsweise durch Destillation. Der im allgemeinen vorhandene Überschuß an Wasserstoff kann in den Reaktor zurückgeführt werden, wobei man vorzugsweise einen kleinen Anteil auschleust.

Die erfindungsgemäße Hydrierung von Phenolen liefert mit den erfindungsgemäßen Trägerkatalysatoren überraschenderweise Produkte, die ganz überwiegend, im allgemeinen zu über 90 Gew.-%, häufig zu über 95 Gew.-%, die entsprechenden Cyclohexanole und nur sehr wenig, im allgemeinen zu weniger als 8 Gew.-%, die entsprechenden Cyclohexanone enthalten. Die Bindung sonstiger Nebenprodukte ist sehr gering, die Standzeiten der einzusetzenden Katalysatoren hoch.

Diese ganz überwiegende Bildung von Cyclohexanolen konnte vor allem deshalb nicht erwartet werden, da gemäß dem Stand der Technik bei der Dotierung von Palladium enthaltenden Katalysatoren genau das Gegenteil eintritt, nämlich auf hohem Niveau noch eine Förderung der Bildung von Cyclohexanonen und auf niedrigem Niveau noch eine weitere Zurückdrängung der Bildung von Cyclohexanolen.

Beispielsweise beschreibt die DE-B-12 98 098 die Herstellung von Cyclohexanon durch Hydrierung von Phenolen in Gegenwart von Katalysatoren, die neben Palladium Erdalkalihydroxide auf $\gamma$-$Al_2$-$O_3$-Trägern enthalten, wobei Gemische mit 88 bis 95 Gew.-% Cyclohexanon und 2 bis 6 Gew.-% Cyclohexanol erhalten werden. Im Gegensatz zur vorliegenden Erfindung wird gemäß der DE-B-12 98 098 allerdings $\gamma$-$Al_2O_3$ als Trägermaterial eingesetzt, d. h. ein Trägermaterial mit einer BET-Oberfläche von ca. 100 bis 300 $m^2$/g.

Die DE-A-27 52 291 beschreibt ebenfalls die Hydrierung von Phenol zu Cyclohexanon. Hier werden Katalysatoren, die aus mit Palladium überzogenen Kohlenstoff- oder Kohleteilchen mit einer (inneren) Oberfläche von 100 bis 2000 $m^2$/g bestehen, und ein Phenol eingesetzt, das eine kleine Menge Alkaliverbindungen enthält. Basierend auf der Wechselwirkung des im Phenol enthaltenen Promotors mit dem Katalysator wird z. B. erreicht, daß nach 210 Minuten Hydrierzeit ein Produkt erhalten wird, das 1,1 Gew.-% Cyclohexanol, 87,5 Gew.-% Cyclohexanon und 11,4 Gew.-% Phenol enthält, während beim Weglassen des Promotors im Einsatzphenol bei sonst gleichen Bedingungen ein Produkt erhalten wird, das 1,4 Gew.-% Cyclohexanol, 65,9 Gew.-% Cyclohexanon und 32,5 Gew.-% Phenol enthält (siehe Beispiel 1 aus der DE-A-27 52 291). Im Gegensatz zur vorliegenden Erfindung wird auch hier ein Träger mit großer innerer Oberfläche eingesetzt, diesmal auf der Basis von Kohle.

Schließlich werden auch in der US-A-3 076 810 Phenolhydrierungen beschrieben. Durch Beispiele belegt ist, daß mit einem Phenol, das auf 1000 Teile 0,01 Teile Natrium-carbonat enthält und mit einem Katalysator, der 5 % Palladium auf Kohle, sowie 5000 ppm Natrium enthält ein Produktgemisch mit der Zusammensetzung 97,2 % Cyclohexanon, unter 0,5 % Phenol, Rest Cyclohexanol erhalten wird (siehe Beispiel 1A). Variationen der Natriummenge im Katalysator ändern den Cyclohexanolgehalt (maximal ca. 3 %) des Produktes nur unwesentlich (siehe Beispiele 1B und 1D). Produkte mit hohem Cyclohexanolanteil (maximal 80 %) werden nur dann erhalten, wenn ein mit Natrium dotierter Palladium-Kohle-Katalysator und ein Phenol eingesetzt wird, das relativ viel Soda enthält (siehe Beispiel 4). Auch dieser Stand der Technik führt von der vorliegenden Erfindung weg, denn er legt nahe, zur Erhöhung der Selektivität für Cyclohexanol das Einsatzphenol zu variieren und nicht den Katalysator. Außerdem werden mit erfindungsgemäßen Katalysatoren noch deutlich höhere Cyclohexanolselektivitäten als 80 % erreicht.

Die nachfolgenden Beispiele dienen zur weiteren Erläuterung der Erfindung, ohne sie in irgendeiner Weise zu beschränken.

(Siehe Tabellen Seite 7ff.)

B) Herstellung der Katalysatoren

a) Allgemeine Beschreibung der Katalysatorherstellung

1000 ml eines Trägers aus Tabelle 1 wurden bei Raumtemperatur mit einer die erforderliche Menge Base enthaltenden Lösung in einer rotierenden Trommel bis zur Sättigung imprägniert. Das Volumen der Imprägnierlösung wurde über die Saugfähigkeit und das Schüttgewicht des Trägers berechnet. Die dementsprechend eingesetzte Imprägnierlösung wurde vom jeweiligen Träger innerhalb weniger Minuten völlig aufgesaugt. Der so imprägnierte Träger wurde in einer rotierenden Trommel oder in einem anderen geeigneten Gefäß in einem warmen, gegebenenfalls inerten Gasstrom bis zu 200 °C bis zur Gewichtskonstanz getrocknet.

Der so vorbehandelte, trockene Träger wurde erneut entsprechend seiner Saugfähigkeit mit einer die erforderliche Menge Palladiumsalze enthaltenden Lösung, wie oben beschrieben, imprägniert. Nach dieser Imprägnierung wurde der feuchte Träger in ein geeignetes verschließbares Gefäß gefüllt und bis zu mehreren Tagen darin belassen. Der so behandelte Träger wurde anschließend mit Wasser alkalifrei

## Beispiele

A) Beschreibung der eingesetzten Trägermaterialien

Tabelle 1

| Träger Nr. | Träger Zusammensetzung | Geometrische Form Form | BET-oberfläche ($m^2/g$) | Schütt-gew. (g/l) | $H_2O$-Aufnahme (ml/100 g) |
|---|---|---|---|---|---|
| 1 | $Al_2O_3$ | Kugeln 3 - 6 mm | 9,8 | 812 | 45,1 |
| 2 | $Al_2O_3$(5 % $SiO_2$ | Extrudat 5 mm ø | 6,0 | 1075 | 26,4 |
| 3 | $Al_2O_3$ | Tabletten 5 x 5 mm | 8,5 | 562 | 85,0 |
| 4 | Al-Silikat ~ 85 % $Al_2O_3$ ~ 15 % $SiO_2$ | Granulat 3 - 5 mm ø | 4,5 | 1190 | 22,4 |

Tabelle 1 (Fortsetzung)

| Träger Nr. | Träger Zusammensetzung | Geometrische Form Form | BET-oberfläche ($m^2/g$) | Schütt-gew. (g/l) | $H_2O$-Aufnahme (ml/100 g) |
|---|---|---|---|---|---|
| 5 | Mg-Al-Silikat<br>- 53 % $Al_2O_3$<br>- 32 % $SiO_2$<br>- 15 % MgO | Kugeln 6 mm ø | 2,1 | 1060 | 21,0 |
| 6 | Al-Silikat<br>- 88 % $SiO_2$<br>- 12 % $Al_2O_3$ | Kugeln 8 mm ø | 1,0 | 1000 | 28,6 |
| 7 | Zr-Mg-Silikat<br>- 88 % $SiO_2$<br>- 6 % $ZrO_2$<br>- 3 % MgO<br>Rest $Al_2O_3$ | Kugeln 8 mm ø | 5,6 | 1050 | 12,4 |
| 8 | $SiO_2$ ( 5 % $Al_2O_3$ | Kugeln 5 mm ø | 10,0 | 834 | 27,0 |
| 9 | $SiO_2$ | Tabletten 3 x 3 mm | 6,5 | 1132 | 11,0 |

gewaschen und getrocknet.

Der so erhaltene Träger wurde entsprechend seiner Saugfähigkeit mit einer die erforderliche Menge Base enthaltenden Lösung, wie oben beschrieben, getränkt und danach bis zur Gewichtskonstanz getrocknet.

b) Beispiel 1

1 l des α-Aluminiumoxidträgers Nr. 1 (siehe Tabelle 1) wurde mit 366 ml einer 10,8 g, entsprechend 0,27 Grammäquivalenten, NaOH enthaltenden wäßrigen Lösung imprägniert. Die Lösung wurde innerhalb weniger Minuten völlig vom Träger aufgenommen. Der feuchte Träger wurde in ein senkrechtes Glasrohr von 2 l Inhalt geschüttet und in einem Warmluftstrom von 120 °C mit einer Luftmenge von 25 $Nm^3$ Luft pro Stunde bis zur Gewichtskonstanz getrocknet.

Der so vorbehandelte trockene Träger wurde seiner Saugfähigkeit entsprechend mit 366 ml einer wäßrigen Natrium-tetrachloropalladat-(II)-Lösung, die 9 g Palladium, entsprenchend 0,169 Grammäquivalenten, enthielt, getränkt und feucht in ein entsprechend dimensioniertes, verschließbares Gefäß umgefüllt.

Die im Träger enthaltene Menge NaOH entsprach einem Äquivalenzverhältnis (g-Äquivalent NaOH : g-Äquivalent Pd) von 1,60. Nach einer Reaktionszeit von 2 Stunden wurde der mit Natriumtetrachloropalladat-(II) imprägnierte, mit Natronlauge vorbehandelte Träger in einem fließenden Strom von destilliertem Wasser gewaschen, bis kein Alkali und keine Ionen der bei seiner Herstellung verwendeten Verbindungen im Waschwasser mehr nachweisbar waren, was nach 10 Stunden der Fall war.

Die anschließende Trocknung wurde im Warmluftstrom vorgenommen wie oben beschrieben.

Der so mit Palladium beladene Katalysatorvorläufer enthielt das Palladium innerhalb einer eng begrenzten, im Inneren und dicht unter der Oberfläche des Trägers liegenden Zone.

Nach dieser Behandlung wurde der palladiumhaltige Katalysatorvorläufer mit 366 ml einer 10,0 g, entsprechend 0,063 Grammäquivalenten Bariumhydroxid-Oktahydrat enthaltenden wäßrigen Tränkflüssigkeit imprägniert und analog der oben beschriebenen Trocknung im Warmluftstrom bei 120 °C getrocknet.

Der Katalysator wurde anschließend im Wasserstoffstrom bei 180 °C reduziert. Der fertige Katalysator enthielt 9 g Palladium und, als wasserfreies Hydroxid berechnet, 5,43 g Bariumhydroxid pro Liter Träger. Die im Katalysator enthaltene Menge Bariumhydroxid entsprach einem Äquivalenzverhältnis (g-Äquivalent Base : g-Äquivalent Palladium) von 0,373.

Beispiel 2

Es wurde verfahren wie in Beispiel 1 beschrieben, jedoch wurde der mit Natriumtetrachloropalladat-(II)-imprägnierte Träger vor dem Waschen mit 400 ml einer wäßrigen, 10 %igen Hydrazinhydratlösung überschichtet und 2 Stunden stehen gelassen, um die auf dem Träger abgeschiedenen Palladiumverbindungen zu metallischem Palladium zu reduzieren. Danach wurde der Palladium enthaltende Träger wie in Beispiel 1 beschrieben gewaschen, getrocknet, mit Bariumhydroxid-Oktahydrat imprägniert, erneut getrocknet und im Wasserstoffstrom behandelt.

Beispiele 3-18

Es wurde verfahren wie in Beispiel 1, jedoch wurde die Menge NaOH, die Menge Palladium und die Menge und Art der nach dem Abscheiden von Palladium aufgebrachten Base variert. Die Einzelheiten sind aus Tabelle 2 ersichtlich.

In Tabelle 2 steht « g-Äqu. » für Gramm-Äquivalente. Die Angaben « $Ba(OH)_2$ » und « $Sr(OH)_2$ » stehen jeweils für $Ba(OH)_2 \times 8H_2O$ und $Sr(OH)_2 \times 8H_2O$. Bei Beispiel 12 lag das $Ca(OH)_2$ zum größten Teil in suspendierter Form vor.

(Siehe Tabelle Seite 10f.)

Beispiel 19 (zum Vergleich) :

Es wurde gemäß der allgemeinen Beschreibung der Katalysatorherstellung verfahren und 1000 ml des Trägers Nr. 1 (siehe Tabelle 1) mit 366 ml einer 21,6 g, entsprechend 0,54 Grammäquivalenten, NaOH enthaltenden wäßrigen Lösung imprägniert, bis zur Gewichtskonstanz getrocknet und anschließend mit 366 ml einer wäßrigen Natriumtetrachloropalladat-(II)-Lösung, die 18 g Palladium, entsprechend 0,338 Grammäquivalent, enthielt, getränkt und in ein verschließbares Gefäß umgefüllt. Nach einer Reaktionszeit von 2 Stunden wurde der Katalysator alkalifrei gewaschen und getrocknet und anschließend mit Wasserstoffgas reduziert. Der Katalysator entsprach somit demjenigen der Beispiele 4 und 7 (siehe Tabelle 2), jedoch ohne die erfindungsgemäße Alkali-Nachbehandlung.

Tabelle 2

| Beispiel Nr. | Katalysatorvorläufer: Träger Nr. (s.Tab.1) | Träger (g) | NaOH (g) | NaOH (g-Äqu.) | Pd in Form v. $Na_2PdCl_4$ (g) | Pd in Form v. $Na_2PdCl_4$ (g-Äqu.) | g-Äqu. NaOH pro g-Äqu. Pd | erfindungsgemäßer Katalysator: erfindungsgemäße Base Art | Base (g) | Base (g-Äqu.) | g-Äqu. Base pro g-Äqu. Pd |
|---|---|---|---|---|---|---|---|---|---|---|---|
| 3 | 1 | 812 | 3,0 | 0,075 | 5,0 | 0,094 | 0,80 | $Ba(OH)_2$ | 10 | 0,063 | 0,670 |
| 4 | 1 | 812 | 21,6 | 0,540 | 18,0 | 0,338 | 1,60 | $Ba(OH)_2$ | 10 | 0,063 | 0,186 |
| 5 | 1 | 812 | 10,8 | 0,270 | 9,0 | 0,169 | 1,60 | $Ba(OH)_2$ | 20 | 0,127 | 0,751 |
| 6 | 1 | 812 | 10,8 | 0,270 | 9,0 | 0,169 | 1,60 | KOH | 10 | 0,178 | 1,053 |
| 7 | 1 | 812 | 21,6 | 0,540 | 18,0 | 0,338 | 1,60 | KOH | 10 | 0,178 | 0,527 |
| 8 | 1 | 812 | 10,8 | 0,270 | 9,0 | 0,169 | 1,60 | KOH | 16 | 0,285 | 1,686 |
| 9 | 1 | 812 | 10,8 | 0,270 | 9,0 | 0,169 | 1,60 | LiOH | 8 | 0,334 | 1,976 |
| 10 | 1 | 812 | 10,8 | 0,270 | 9,0 | 0,169 | 1,60 | MgO | 10 | 0,496 | 2,935 |
| 11 | 2 | 1075 | 14,4 | 0,360 | 12,0 | 0,225 | 1,60 | $Sr(OH)_2$ | 10 | 0,075 | 0,333 |
| 12 | 3 | 562 | 21,6 | 0,540 | 9,0 | 0,169 | 3,20 | $Ca(OH)_2$ | 10 | 0,270 | 1,598 |
| 13 | 4 | 1190 | 43,2 | 1,080 | 9,0 | 0,169 | 6,39 | $Ba(OH)_2$ | 10 | 0,063 | 0,373 |
| 14 | 5 | 1060 | 21,6 | 0,540 | 9,0 | 0,169 | 3,20 | $Ba(OH)_2$ | 10 | 0,063 | 0,373 |
| 15 | 6 | 1000 | 10,8 | 0,270 | 9,0 | 0,169 | 1,60 | $Ba(OH)_2$ | 10 | 0,063 | 0,373 |
| 16 | 7 | 1050 | 8,1 | 0,203 | 9,0 | 0,169 | 1,20 | $Ba(OH)_2$ | 10 | 0,063 | 0,373 |
| 17 | 8 | 834 | 8,1 | 0,203 | 9,0 | 0,169 | 1,20 | $Ba(OH)_2$ | 10 | 0,063 | 0,373 |
| 18 | 9 | 1132 | 5,4 | 0,135 | 9,0 | 0,169 | 0,80 | $Ba(OH)_2$ | 10 | 0,063 | 0,373 |

Beispiel 20 (zum Vergleich) :

1000 ml eines in der DE-A-2 045 882 im Beispiel 1 beschriebenen Lithium-Aluminium-Spinell-Trägers mit einer BET-Oberfläche von 25 $m^2/g$ wurden mit einem seiner Saugfähigkeit entsprechenden Volumen einer wäßrigen Natriumtetrachloropalladat-(II)-Lösung, die 18 g Palladium enthielt, getränkt und in einem Becherglas mit 400 ml einer wäßrigen, 10 %igen Hydrazinhydrat enthaltenden Lösung überschichtet. Nach einer Reaktionszeit von 2 Studen wurde der Katalysator in einem fließenden Strom von destilliertem Wasser gewaschen und getrocknet.

Der so hergestellte Palladium enthaltende Katalysator enthielt das Palladium in einer unregelmäßigen Verteilung in den einzelnen Katalysatorkugeln, wobei alle Übergänge zwischen ringförmiger Abscheidungen an der Katalysatoroberfläche bis zur völligen Durchtränkung bis ins Kugelinnere auftraten.

Nach der Trocknung wurde der Palladium enthaltende Katalysator mit dem seiner Saugfähigkeit entsprechenden Volumen einer wäßrigen, 10 g KOH enthaltenden Lösung imprägniert und getrocknet.

Der fertige Katalysator enthielt 18 g Palladium und 10 g KOH pro Liter Li-Al Spinell-Träger. Die im Katalysator enthaltene Menge KOH entsprach einem Äquivalenzverhältnis (g-Äquivalent Base : g-Äquivalent Palladium) von 0,527.

Beispiele 21-34

Ein Einrohr-Hydrierreaktor (Rohrdurchmesser 4 cm, Rohrlänge 80 cm) wurde jeweils mit einem Liter Katalysator befüllt. Die Aktivierung des Katalysators wurde im Wasserstoffstrom bei einer Temperatur von 180 °C vorgenommen. Der Reaktor wurde anschließend auf eine Temperatur zwischen 130 und 170 °C eingestellt. Nachdem diese erreicht war, wurde Phenol bei einer Temperatur von ca. 160 °C in den Wasserstoffstrom hineinverdampft. Das Phenol-Wasserstoff-Gemisch (Mol-Verhältnis Phenol : Wasserstoff = 1 : 6 bis 1 : 15) durchströmte den Reaktor von unten nach oben. Die Belastung wurde bei 0,3 bis 0,5 l Phenol pro Stunde und pro Liter Katalysator gehalten. Das gasförmige Reaktions produkt wurde nach dem Verlassen des Reaktors kondensiert. Die Zusammensetzung des Produktgemisches wurde jeweils nach 24 Stunden Laufzeit bestimmt. Die Einzelheiten sind aus Tabelle 3 ersichtlich.

In der Tabelle 3 ist in der Spalte « Katalysatorstandzeit » derjenige Zeitraum angegeben, innerhalb dem das Produktgemisch über 90 Gew.-% Cyclohexanol enthielt. Bei den Beispielen 33 und 34 handelt es sich um Vergleichsbeispiele.

(Siehe Tabelle Seite 12f.)

Beispiel 35

Es wurde verfahren wie allgemein für die Beispiele 21 bis 34 beschrieben, jedoch wurde anstelle von Phenol m-Kresol eingesetzt. Als Katalysator wurde derjenige des Beispiel 1 eingesetzt. Die Reaktionstemperatur betrug 150 °C, die Belastung 0,3 l m-Kresol pro Stunde und pro Liter Katalysator und das Molverhältnis m-Kresol : Wasserstoff betrug 1 : 10. Nach 20 Stunden Laufzeit wurde die Zusammensetzung des Produktgemisches bestimmt. Es enthielt 92,2 Gew.-% Methylcyclohexanol, 7,0 Gew.-% Methylcyclohexanon und 0,8 Gew.-% Nebenprodukte. Die Standzeit des Katalysators, d. h. die Zeit bis der Gehalt des Reaktionsproduktes an Methylcyclohexanol auf unter 90 % gefallen war, betrug 730 Stunden.

Beispiel 36 (zum Vergleich) :

Es wurde verfahren wie in Beispiel 35, jedoch wurde als Katalysator der nicht mit Base nachbehandelte Katalysator aus Beispiel 19 eingesetzt. Nach 20 Stunden enthielt das Produktgemisch 31,7 Gew.-% Methylcyclohexanol, 67,8 Gew.-% Methylcyclohexanon und 0,5 Gew.-% Nebenprodukte.

Tabelle 3

| Beispiel Nr. | Einsatzmaterialien: Katalysator aus Beisp. Nr. | Phenol (l/h) | Wasserstoff (Mol pro Mol Phenol) | Reaktions-temperatur (°C) | Produktzusammensetzung: Cyclohexanol (Gew.-%) | Cyclohexanon (Gew.-%) | Nebenprodukte (Gew.-%) | Katalysator-standzeit (h) |
|---|---|---|---|---|---|---|---|---|
| 21 | 1 | 0,3 | 10,5 | 130 | 98,8 | 1,1 | 0,1 | 1270 |
| 22 | 2 | 0,3 | 10,5 | 130 | 94,7 | 4,7 | 0,6 | 740 |
| 23 | 3 | 0,3 | 10,5 | 160 | 93,8 | 5,8 | 0,4 | 520 |
| 24 | 4 | 0,3 | 10,5 | 140 | 98,4 | 1,1 | 0,5 | 1120 |
| 25 | 5 | 0,3 | 11,0 | 140 | 98,2 | 1,6 | 0,2 | 1180 |
| 26 | 6 | 0,5 | 10,5 | 130 | 99,0 | 0,9 | 0,1 | 630 |
| 27 | 7 | 0,3 | 9,0 | 150 | 96,0 | 3,8 | 0,2 | 610 |
| 28 | 8 | 0,4 | 11,0 | 130 | 98,1 | 1,6 | 0,3 | 480 |
| 29 | 9 | 0,3 | 10,5 | 140 | 97,9 | 2,0 | 0,1 | 1060 |
| 30 | 10 | 0,3 | 10,5 | 130 | 94,3 | 5,2 | 0,5 | 240 |
| 31 | 11 | 0,3 | 11,0 | 140 | 95,2 | 4,5 | 0,3 | 550 |
| 32 | 12 | 0,2 | 10,0 | 170 | 93,9 | 5,5 | 0,6 | 520 |
| 33 | 19 | 0,3 | 10,5 | 140 | 75,5 | 23,7 | 0,8 | - |
| 34 | 20 | 0,3 | 11,0 | 140 | 14,1 | 79,4 | 6,5 | - |

**Patentansprüche**

1. Trägerkatalysatoren, erhältlich indem man nacheinander

a) ein inertes Trägermaterial mit einer BET-Oberfläche von weniger als 20 $m^2/g$ mit einer Base bis zur Sättigung tränkt,

b) bis zur Gewichtskonstanz trocknet

c) mit einer Palladiumsalzlösung bis zur Sättigung tränkt,

d) mit Wasser wäscht, bis keine Ionen aus den bis jetzt verwendeten Verbindungen mehr im Waschwasser nachweisbar sind und

e) trocknet,

dadurch gekennzeichnet, daß man danach

f) so viel einer Base aufbringt, daß der Katalysator 0,01 bis 50 Grammäquivalente Base pro Grammäquivalent Palladium enthält,

g) bis zur gewichtskonstanz trocknet und

h) zu einem beliebigen Zeitpunkt nach Durchführung des Schrittes c) und vor der Durchführung der mit dem Katalysator zu katalysierenden Reaktion die abgeschiedenen Palladiumverbindungen zum Metall reduziert, bei Durchführung dieser Reduktion nach dem Schritt f) unter Bedingungen, bei denen die im Schritt f) aufgebrachte Base auf dem Katalysator verbleibt.

2. Trägerkatalysatoren gemäß Anspruch 1, dadurch gekennzeichnet, daß sie erhältlich sind, indem man in Stufe a) als Trägermaterial Aluminiumoxide, Siliciumdioxide, Siliciumdioxid-Aluminiumoxid-Gemische, amorphe Kieselsäuren, Kieselgure, Barium-, Strontium-oder Calciumcarbonate, Mischungen derselben, die gegebenenfalls zusätzlich Siliciumdioxide oder Aluminiumoxide enthalten, Titanoxide, Zirkonoxide, Magnesiumoxide, Magnesiumsilikate, Zirkonsilikate, Magnesium-Aluminium-Spinelle, Siliciumcarbide, Wolframcarbide, Mischungen von Siliciumcarbiden mit Siliciumdioxiden oder beliebige Mischungen der vorgenannten Materialien und als Base ein Oxid, Carbonat, Hydrogencarbonat, Hydrogenphosphat, Hydroxid, Alkoxid, Formiat, Alkalisilikat, Alkalialuminat oder Mischungen davon verwendet und in Stufe c) eine wäßrige Lösung von $Na_2[PdCl_4]$, $PdCl_2$ und/oder $Pd(NO_3)_2$ in einer Konzentration verwendet, daß mit einer einmaligen Tränkung 1 bis 20 g Palladium auf den Träger aufgebracht werden.

3. Trägerkatalysatoren gemäß Ansprüchen 1 und 2, dadurch gekennzeichnet, daß sie erhältlich sind, indem man in Stufe f) Oxide, Hydroxide, Alkoxide, Carbonate, Hydrogencarbonate, Hydrogenphosphate, Formiate, Silikate und/oder Aluminate von Alkali- und/oder Erdalkalimetallen aufbringt.

4. Trägerkatalysatoren gemäß Ansprüchen 1 bis 3, dadurch gekennzeichnet, daß sie erhältlich sind, indem man im Falle der Durchführung der Stufe h) vor der Stufe f) mit Hydrazinhydrat, Wasserstoff, Formaldehyd oder Natriumborhydrid und im Falle der Durchführung der Stufe h) nach der Stufe f) mit gasförmigem Wasserstoff reduziert.

5. Verfahren zur Herstellung von Trägerkatalysatoren, bein dem man nacheinander

a) ein inertes Trägermaterial mit einer BET-Oberfläche von weniger als 20 $m^2/g$ mit einer Base bis zur Sättigung tränkt,

b) bis zur Gewichtskonstanz trocknet,

c) mit einer Palladiumsalzlösung bis zur Sättigung tränkt,

d) mit Wasser wäscht, bis keine Ionen aus den bis jetzt verwendeten Verbindungen mehr im Waschwasser nachweisbar sind und

e) trocknet,

dadurch gekennzeichnet, daß man danach

f) so viel einer Base aufbringt, daß der Katalysator 0,01 bis 50 Grammäquivalente Base pro Grammäquivalent Palladium enthält,

g) bis zur Gewichtskonstanz trocknet und

h) zu einem beliebigen Zeitpunkt nach Durchführung des Schrittes c) und vor der Durchführung der mit dem Katalysator zu katalysierenden Reaktion die abgeschiedenen Palladiumverbindungen zum Metall reduziert, bei der Durchführung dieser Reduktion nach dem Schritt f) unter Bedingungen, bei denen die im Schritt f) aufgebrachte Base auf dem Katalysator verbleibt.

6. Verfahren zur Hydrierung von gegebenenfalls substituierten Phenolen zu Cyclohexanolen, dadurch gekennzeichnet, daß man es in Gegenwart von Trägerkatalysatoren durchführt, die erhältlich sind, indem man nacheineinander

a) ein inertes Trägermaterial mit einer BET-Oberfläche von weniger 20 $m^2/g$ mit einer Base bis zur Sättigung tränkt,

b) bis zur Gewichtskonstanz trocknet,

c) mit einer Palladiumsalzlösung bis zur Sättigung tränkt,

d) mit Wasser wäscht, bis keine Ionen aus den bis jetzt verwendeten Verbindungen mehr im Waschwasser nachweisbar sind,

e) trocknet,

f) so viel einer Base aufbringt, daß der Katalysator 0,01 bis 50 Grammäquivalente Base pro Grammäquivalent Palladium enthält,

g) bis zur Gewichtskonstanz trocknet und

h) zu einem beliebigen Zeitpunkt nach Durchführung des Schrittes c) und vor der Durchführung der mit dem Katalysator zu katalysierenden Reaktion die abgeschiedenen Palladiumverbindungen zum Metall reduziert, bei der Durchführung dieser Reduktion nach dem Schritt f) unter Bedingungen, bei denen die im Schritt f) aufgebrachte Base auf dem Katalysator verbleibt.

7. Verfahren nach Anspruch 6, dadurch gekennzeichnet, daß man ein Phenol der Formel (I) einsetzt

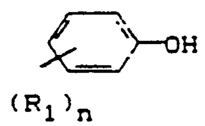

(I)

in der

$R_1$ für $C_1$-bis $C_6$-Alkyl, $C_6$- bis $C_{10}$-Aryl, Hydroxyl, Nitro, Halogen, gegebenenfalls substituiertes Amino, $C_1$- bis $C_6$-Alkoxy oder $C_6$-bis $C_{10}$-Aroxy und

n für Null oder eine ganze Zahl von 1 bis 5 stehen, wobei jedoch im Falle $R_1$ = Nitro n nur für Null, 1 oder 2 steht oder ein Phenol der Formel (II) einsetzt

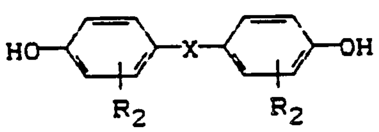

(II)

in der

$R_2$ unabhängig voneinander jeweils für Wasserstoff, $C_1$- bis $C_6$-Alkyl, $C_1$- bis $C_6$-Alkoxy oder Halogen und

X für $—CH_2—$, $—C(CH_3)_2—$, -Cyclohexyl-, —CO—, —S—, $—SO_2—$, —O— oder eine Einfachbindung stehen.

8. Verfahren nach Anspruch 7, dadurch gekennzeichnet, daß man ein Phenol der Formel (I) einsetzt, in der $R_1$ für Methyl, Ethyl, n-Propyl, i-Propyl, n-Hexyl, Phenyl, Hydroxyl, Chlor, Brom, $NH_2$, Methoxy, Ethoxy oder Phenoxy und n für Null, 1 oder 2 stehen.

9. Verfahren nach Ansprüchen 6 bis 8, dadurch gekennzeichnet, daß man es in Gegenwart von Katalysatoren durchführt, die erhältlich sind, indem man in Stufe a) als Trägermaterial $\alpha$-$Al_2O_3$ und in Stufe f) als Base Bariumhydroxid einsetzt.

10. Verfahren nach Ansprüchen 6 bis 9, dadurch gekennzeichnet, daß man es bei Temperaturen zwischen 100 und 350 °C und Drucken zwischen 1 und 350 bar durchführt.

## Claims

1. Supported catalysts which can be obtained by, successively :

a) impregnating an inert supporting material having a BET surface area less than 20 m²/g with a base until saturation is reached,

b) drying the material to constant weight,

c) impregnating it with a palladium salt solution until saturation is reached,

d) washing it with water until ions from the compounds used up to this point can no longer be detected in the wash water, and

e) drying,

characterized in that, thereafter :

f) a base is applied in a quantity such that the catalyst contains 0.01 to 50 gram equivalents of base per gram equivalent of palladium,

g) the catalyst is dried to constant weight and

h) the deposited palladium compounds are reduced to the metal at any desired point in time after carrying out stage c) and before carrying out the reaction to be catalyzed by means of the catalyst, and, if carrying out this reduction after stage f), doing so under conditions in which the base applied in stage f) remains on the catalyst.

2. Supported catalysts according to Claim 1, characterized in that they can be obtained by using aluminium oxides, silicon dioxides, silicon dioxide/aluminium oxide mixtures, amorphous silicas, kieselguhrs, barium, strontium or calcium carbonates, mixtures thereof optionally containing, in addition, silicon dioxides or aluminium oxides, titanium oxides, zirconium oxides, magnesium oxides, magnesium silicates, zirconium silicates, magnesium/aluminium spinels, silicon carbides, tungsten carbides, mixtures of silicon carbides with silicon dioxides or any desired mixtures of the abovementioned materials as the supporting material, and an oxide, carbonate, bicarbonate, hydrogen phosphate, hydroxide, alkoxide, formate, alkali metal silicate, alkali metal aluminate or mixtures thereof as the base, in stage a), and using,

in stage c), an aqueous solution of $Na_2[PdCl_4]$, $PdCl_2$ and/or $Pd(NO_3)_2$ in a concentration such that 1 to 20 g of palladium are applied to the support by means of a single impregnation.

3. Supported catalysts according to Claims 1 and 2, characterized in that they can be obtained by applying oxides, hydroxides, alkoxides, carbonates bicarbonates, hydrogenphosphates, formates, silicates and/or aluminates of alkali and/or alkaline earth metals in stage f).

4. Supported catalysts according to Claims 1 to 3, characterized in that they can be obtained by carrying out reduction with hydrazine hydrate, hydrogen, formaldehyde or sodium borohydride in the event that stage h) is carried out before stage f), and carrying out reduction by means of gaseous hydrogen in the event that stage h) is carried out after stage f).

5. Process for the preparation of supported catalysts, in which, successively :

a) an inert supporting material having a BET surface area less than 20 $m^2/g$ is impregnated to saturation with a base,

b) is dried to constant weight,

c) is impregnated to saturation with a palladium slat solution,

d) is washed with water until ions from the compounds used up to this point can no longer be detected in the wash water and

e) is dried,

characterized in that, thereafter

f) a base is applied in a quantity such that the catalyst contains 0.01 to 50 gram equivalents of base per gram equivalent of palladium,

g) the catalyst is dried to constant weight and

h) the deposited palladium compounds are reduced to the metal at any desired point in time after carrying out stage c) and before carrying out the reaction to be catalyzed by means of the catalyst, if this reduction is carried out after stage f), it is carried out under conditions in which the base applied in stage f) remains on the catalyst.

6. Process for the hydrogenation of optionally substituted phenols to give cyclohexanols, characterized in that it is carried out in the presence of supported catalysts which can be obtained by, successively :

a) impregnating an inert supporting material having a BET surface area less than 20 $m^2/g$ with a base until saturation is reached,

b) drying the material to constant weight,

c) impregnating it with a palladium salt solution until saturation is reached,

d) washing it with water until ions from the compounds used up to this point can no longer be detected in the wash water,

e) drying,

f) applying a base in a quantity such that the catalyst contains 0.01 to 50 gram equivalents of base per gram equivalent of palladium,

g) drying to constant weight and

h) reducing the deposited palladium compounds to the metal at any desired point in time after carrying out stage c) and before carrying out the reaction to be catalyzed by means of the catalyst, and, if carrying out this reduction after stage f), doing so under conditions in which the base applied in stage f) remains on the catalyst.

7. Process according to Claim 6, characterized in that a phenol of the formula (I)

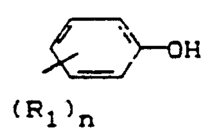

(I)

in which

$R_1$ represents $C_1$ to $C_6$ alkyl, $C_6$ to $C_{10}$ aryl, hydroxyl, nitro, halogen, optionally substituted amino, $C_1$ to $C_6$ alkoxy or $C_6$ to $C_{10}$ aroxy and n represents zero or an integer from 1 to 5, but, in the event that $R_1$ = nitro, n only represents zero, 1 or 2, or a phenol of the formula (II)

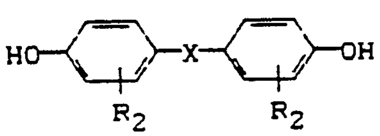

(II)

in which the

$R_2$s independently of one another each represent hydrogen, $C_1$ to $C_6$ alkyl, $C_1$ to $C_6$ alkoxy or halogen and

X represents $-CH_2-$, $-C(CH_3)_2-$, -cyclohexyl-, $-CO-$, $-S-$, $-SO_2-$, $-O-$ or a single bond, is employed.

8. Process according to Claim 7, characterized in that a phenol of the formula (I) in which $R_1$ represents methyl, ethyl, n-propyl, i-propyl, n-hexyl, phenyl, hydroxyl, chlorine, bromine, $NH_2$, methoxy, ethoxy or phenoxy and n represents zero, 1 or 2, is employed.

9. Process according to Claims 6 to 8, characterized in that it is carried out in the presence of catalysts which can be obtained by employing $\alpha$-$Al_2O_3$ as the supporting material in stage a) and barium hydroxide as the base in stage f).

10. Process according to Claims 6 to 9, characterized in that it is carried out at temperatures between 100 and 350 °C and pressures between 1 and 350 bar.

**Revendications**

1. Catalyseurs sur support, obtenus par des opérations successives consistant

a) à imprégner d'une base jusqu'à saturation une matière de support inerte ayant une surface BET de moins de 20 $m^2/g$,

b) à la sécher jusqu'à poids constant,

c) à l'imprégner d'une solution de sel de palladium jusqu'à saturation,

d) à la laver à l'eau jusqu'à ce que des ions provenant des composés utilisés jusqu'ici ne puissent plus être décelés dans l'eau de lavage et

e) à la sécher,

caractérisés en ce qu'on conduit ensuite des opérations qui consistent

f) à apporter une quantité suffisante d'une base pour que les catalyseurs contiennent 0,01 à 50 équivalents en grammes de base par équivalent en grammes de palladium,

g) à sécher jusqu'à poids constant et

h) à réduire en métal les composés de palladium qui se sont déposés, à un moment quelconque après la conduite de l'opération c) et avant la conduite de la réaction devant être catalysée par le catalyseur, en opérant lors de la conduite de cette réduction après l'étape f) dans des conditions telles que la base apportée dans l'étape f) reste sur le catalyseur.

2. Catalyseurs sur support suivant la revendication 1, caractérisés en ce qu'ils sont obtenus par le fait qu'on utilise dans l'étape a), comme matière de support, des oxydes d'aluminium, des dioxydes de silicium, des mélanges de dioxydes de silicium et d'oxyde d'aluminium, des acides siliciques amorphes, des kieselguhrs, des carbonates de baryum, strontium ou calcium, leurs mélanges additionnés le cas échéant de dioxydes de silicium ou d'oxydes d'aluminium, des oxydes de titane, des oxydes de zirconium, des oxydes de magnésium, des silicates de magnésium, des silicates de zirconium, des spinelles de magnésium et d'aluminium, des carbures de silicium, des carbures de tungstène ou des mélanges de carbures de silicium avec des dioxydes de silicium ou des mélanges quelconques des matières mentionnées ci-dessus et, comme base, un oxyde, carbonate, bicarbonate, phosphate acide, hydroxyde, alcoolate, formiate, un silicate alcalin, un aluminate alcalin ou des mélanges de ces composés et dans l'étape c), on utilise une solution aqueuse de $Na_2[PdCl_4]$, $PdCl_2$ et/ou $Pd(NO_3)_2$ à une concentration choisie de manière qu'une quantité de 1 à 20 g de palladium soit appliquée sur le support en une seule imprégnation.

3. Catalyseurs sur support suivant les revendications 1 et 2, caractérisés en ce qu'on les obtient en appliquant dans l'étape f) des oxydes, des hydroxydes, des alcoolates, des carbonates, des bicarbonates, des hydrogénophosphates, des formiates, des silicates et/ou des aluminates de métaux alcalins et/ou de métaux alcalino-terreux.

4. Catalyseurs sur support suivant les revendications 1 à 3, caractérisés en ce qu'on les obtient en effectuant la réduction, dans le cas de la conduite de l'étape h) avant l'étape f), avec de l'hydrate d'hydrazine, de l'hydrogène, du formaldéhyde ou du borohydrure de sodium, et dans le cas de la conduite de l'étape h) après l'étape f), avec l'hydrogène gazeux.

5. Procédé de production de catalyseurs sur support, dans lequel on conduit les opérations successives qui consistent

a) à imprégner d'une base jusqu'à saturation une matière de support inerte de surface BET inférieure à 20 $m^2/g$,

b) à sécher la matière jusqu'à poids constant,

c) à l'imprégner d'une solution de sel de palladium jusqu'à saturation,

d) à la laver à l'eau jusqu'à ce que des ions provenant des composés utilisés jusqu'ici ne puissent plus être décelés dans l'eau de lavage et

e) à la sécher,

caractérisé en ce qu'on conduit ensuite les opérations consistant

f) à apporter une quantité suffisante d'une base pour que le catalyseur contienne 0,01 à 50 équivalents en grammes par équivalent en grammes de palladium,

g) à sécher jusqu'à poids constant et

h) à réduire en métal les composés du palladium qui ont été déposés à un moment quelconque

après la conduite de l'étape c) et avant la conduite de la réaction devant être catalysée par le catalyseur, en opérant dans le cas de la conduite de cette réduction après l'étape f) dans des conditions dans lesquelles la base apportée dans l'étape f) reste sur le catalyseur.

6. Procédé d'hydrogénation en cyclohexanols de phénols éventuellement substitués, caractérisé en ce qu'on opère en présence de catalyseurs sur support que l'on obtient en conduisant des opérations successives consistant

a) à imprégner d'une base jusqu'à saturation une matière de support inerte de surface BET inférieure à 20 $m^2/g$,

b) à la sécher jusqu'à poids constant,

c) à l'imprégner d'une solution de sel de palladium jusqu'à saturation,

d) à la laver avec de l'eau jusqu'à ce que des ions provenant des composés utilisés jusqu'ici ne puissent plus être décelés dans l'eau de lavage,

e) à la sécher,

f) à apporter une quantité suffisante de base pour que le catalyseur contiennent 0,01 à 50 équivalents en grammes de base par équivalent en grammes de palladium,

g) à sécher le palladium jusqu'à poids constant et

h) à réduire en métal les composés du palladium qui ont été déposés, à un moment quelconque après la conduite de l'opération c) et avant la conduite de la réaction devant être catalysée par le catalyseur, en opérant lors de la conduite de cette réduction après l'étape f) dans des conditions telles que la base apportée dans l'étape f) reste sur le catalyseur.

7. Procédé suivant la revendication 6, caractérisé en ce qu'on utilise un phénol de formule (I)

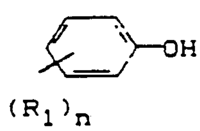

(I)

dans lequelle

$R_1$ représente un groupe alkyle en $C_1$ à $C_6$, un groupe aryle en $C_6$ à $C_{10}$, un groupe hydroxyle, un groupe nitro, un halogène, un groupe amino éventuellement substitué, un groupe alkoxy en $C_1$ à $C_6$ ou un groupe aroxy en $C_6$ à $C_{10}$ et

n est égal à zéro ou à un nombre entier de 1 à 5, sous réserve toutefois que lorsque $R_1$ est un groupe nitro, n n'est que la valeur zéro, 1 ou 2, ou on utilise un phénol de formule (II)

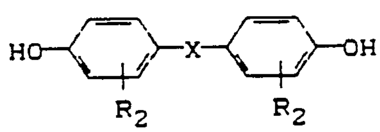

(II)

dans laquelle

les groupes $R_2$ représentent chacun, indépendamment l'un de l'autre, de l'hydrogène, un groupe alkyle en $C_1$ à $C_6$, alkoxy en $C_1$ à $C_6$ ou un halogène et X représente $—CH_2—$, $—C(CH_3)_2—$, -cyclohexyle-, —CO—, —S—, $—SO_2—$, —O— ou une liaison simple.

8. Procédé suivant la revendication 7, caractérisé en ce qu'on utilise un phénol de formule (I) dans laquelle $R_1$ est un groupe méthyle, éthyle, n-propyle, isopropyle, n-hexyle, phényle, hydroxyle, le chlore, le brome, un groupe $NH_2$, méthoxy, éthoxy ou phénoxy et n a la valeur zéro, 1 ou 2.

9. Procédé suivant les revendications 6 à 8, caractérisé en ce qu'on opère en présence de catalyseurs que l'on peut obtenir en utilisant de l'alumine α comme matière de support dans l'étape a) et de l'hydroxyde de baryum comme base dans l'étape f).

10. Procédé suivant les revendications 6 à 9, caractérisé en ce qu'on opère à des températures comprises entre 100 et 350 °C et à des pressions comprises entre 1 et 350 bars.